(19) **Europäisches Patentamt / European Patent Office / Office européen des brevets**

(11) **EP 4 706 664 A1**

(12) # EUROPEAN PATENT APPLICATION
published in accordance with Art. 153(4) EPC

(43) Date of publication:
**11.03.2026 Bulletin 2026/11**

(21) Application number: **24797425.6**

(22) Date of filing: **25.04.2024**

(51) International Patent Classification (IPC):
*A61K 31/713* (2006.01)     *A61P 35/04* (2006.01)
*A23L 33/13* (2016.01)     *G01N 33/50* (2006.01)
*C12Q 1/6886* (2018.01)     *A61K 48/00* (2006.01)

(52) Cooperative Patent Classification (CPC):
**A23L 33/13; A61K 31/713; A61K 48/00;**
**A61P 35/04; C12Q 1/6886; G01N 33/50**

(86) International application number:
**PCT/KR2024/005599**

(87) International publication number:
**WO 2024/225771 (31.10.2024 Gazette 2024/44)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **27.04.2023 KR 20230055666**
**22.04.2024 KR 20240053321**

(71) Applicant: **Ajou University Industry-Academic**
**Cooperation**
**Foundation**
**Suwon-si, Gyeonggi-do 16499 (KR)**

(72) Inventors:
• **EUN, Jung Woo**
**Suwon-si Gyeonggi-do 16223 (KR)**

• **CHEONG, Jae Youn**
**Suwon-si Gyeonggi-do 16504 (KR)**
• **KIM, Soon Sun**
**Suwon-si Gyeonggi-do 16517 (KR)**
• **CHO, Hyo Jung**
**Suwon-si Gyeonggi-do 16493 (KR)**
• **BAEK, Geum Ok**
**Hwaseong-si Gyeonggi-do 18430 (KR)**
• **YOON, Moon Gyeong**
**Suwon-si Gyeonggi-do 16397 (KR)**
• **YOON, Jung Hwan**
**Seoul 06581 (KR)**
• **KIM, Hyung Seok**
**Busan 49459 (KR)**

(74) Representative: **Mewburn Ellis LLP**
**Aurora Building**
**Counterslip**
**Bristol BS1 6BX (GB)**

(54) **COMPOSITION INCLUDING GLUP1 INHIBITOR AS ACTIVE INGREDIENT FOR PREVENTION OR TREATMENT OF RECURRENT LIVER CANCER**

(57)     The present invention relates to a composition including a GULP1 inhibitor as an active ingredient for the prevention or treatment of recurrent liver cancer. GULP1 associated with liver cancer recurrence was derived, and it was confirmed that selective inhibition of GULP1 effectively suppresses the proliferation, migration, and metastasis of liver cancer, particularly preventing the recurrence of liver cancer. As for the mechanism, the inhibition of GULP1 leads to interference with the binding of GULP1 to ARF6, thereby reducing the protein stability of ARF6-GTP and suppressing ARF6 activation, which in turn inhibits β-catenin activity, ultimately controlling the progression of EMT. Therefore, when used, the GULP1 inhibitor can prevent the progression and recurrence of liver cancer and thus may be as a highly promising new therapeutic option for liver cancer.

[FIG. 11]

**Description**

Technical Field

The present disclosure relates to a composition for preventing or treating a recurrent liver cancer, including a GULP1 inhibitor as an active ingredient.

Background Art

According to the report from National Cancer Information Center in 2021, a relative survival rate of liver cancer has been only 37.7% over the past five years, showing the lowest survival rate along with lung cancer (34.7%), with only half the overall cancer survival rate of 70.7%. Even when radioembolization or hepatectomy is involved, the cumulative recurrence rate over two years is 50.0% and 58.3%, respectively. Therefore, understanding the mechanism of recurrence is urgently needed to increase the survival rate of liver cancer patients, as recurrence of liver cancer drops the success rate of liver resection and increases resistance to chemotherapy to result in reduction in the effectiveness of treatment.

Since there is currently no known RNA marker for recurrence, which is a major factor in the survival rate of liver cancer patients, it is essential to discover candidate substances related to liver cancer recurrence and study mechanisms thereof. The technology to suppress gene expression has been an important tool in the development of therapeutics and target validation for the treatment of cancer, and RNA interference (RNAi), since the discovery of its role, acts on sequence-specific mRNAs within cells.

RNAi is a phenomenon in which small interfering RNA (siRNA), a double-stranded ribonucleic acid with a size of 21-25 nucleotides, specifically binds to a transcript with a complementary sequence and degrades the transcript to lead to suppression of expression of a specific protein, where the double-stranded RNA is processed by an endonuclease called Dicer in the cell and converted into double-stranded siRNA which then binds to an RNA-induced silencing complex (RISC) to sequence-specifically inhibit expression of a target gene through degradation as the guide (antisense) strand recognizes target mRNA.

Recently, this ribonucleic acid-mediated interference has been actively studied to be used for development of therapeutics for various diseases, especially tumor treatments, by selectively suppressing the expression of specific proteins at a transcriptome level, as it proposed a solution to problems arising in the development of conventional chemical synthetic drugs, such that it may be a new strategy for preventing liver cancer recurrence and developing treatments.

Disclosure of the Invention

Technical Goals

An object of the present disclosure is to provide a composition for preventing, improving, or treating liver cancer recurrence or liver cancer metastasis, including an inhibitor of GULP1 expression or activity as an active ingredient.

In addition, another object of the present disclosure is to provide a method of screening a treatment agent for liver cancer recurrence or liver cancer metastasis by measuring an expression level of GULP1 in isolated liver cancer cells.

Technical Solutions

To achieve the above objects, the present disclosure provides a pharmaceutical composition for preventing or treating liver cancer recurrence or liver cancer metastasis, including an inhibitor of GULP1 expression or activity as an active ingredient.

In addition, the present disclosure provides a health functional food composition for preventing or improving liver cancer recurrence or liver cancer metastasis, including an inhibitor of GULP1 expression or activity as an active ingredient.

In addition, the present disclosure provides a method of screening a treatment agent for liver cancer recurrence or liver cancer metastasis, including (1) contacting a test substance with isolated liver cancer cells; (2) measuring an expression or activity level of GULP1 in liver cancer cells that have come into contact with the test substance; and (3) selecting a test substance with the expression or activity level of GULP1 reduced compared to a control sample.

Advantageous Effects

The present disclosure relates to a composition for preventing or treating a recurrent liver cancer including a GULP1 inhibitor as an active ingredient, wherein it was confirmed that the composition effectively inhibits proliferation, migration, and metastasis of a liver cancer through selective inhibition of GULP1 by inducing GULP1 related to liver cancer recurrence, and in particular, it is able to prevent liver cancer recurrence, the mechanism of which involves inhibition of binding between GULP1 and ARF6 through suppression of GULP1 that leads to reduction in protein stability of ARF6-GTP and suppression of β-catenin activity by interfering with activation of ARF6, thereby controlling the progression of EMT consequently, such that it is highly likely to be utilized as a new treatment agent for a liver cancer since it is possible to prevent progression and recurrence of the liver cancer using the inhibitor of GULP 1.

Brief Description of Drawings

FIG. 1 shows results of GULP1 expression in three genome datasets.
FIG. 2 shows a result of analyzing expression of GULP1 in 33 carcinomas.
FIG. 3 shows results of survival rate following expression of GULP1.
FIG. 4 shows results of an inhibitory effect on liver cancer proliferation (liver cancer cell growth) following GULP1 inhibition in liver cancer cell lines.
FIG. 5 shows results of an inhibitory effect on liver cancer proliferation (colony forming ability) following GULP1 inhibition in liver cancer cell lines.
FIG. 6 shows results of an inhibitory effect on a healing ability for liver cancer following GULP1 inhibition in liver cancer cell lines.
FIG. 7 shows results of identifying a cell sphere forming ability and metastatic ability by GULP1.
FIG. 8 shows results of detecting translocation of GULP1 mediated by β-catenin.
FIG. 9 shows results of detecting translocation of β-catenin mediated by ARF6.
FIGS. 10 and 11 show results of detecting attachment of ARF6-mediated β-catenin to a promoter region of GULP1.
FIG. 12 shows results of regulating downstream factors of GULP1.
FIGS. 13 and 14 show results of identifying a tumor formation inhibitory ability by suppression of GULP1.
FIG. 15 shows results of identifying an inhibitory ability on liver cancer recurrence by suppression of GULP 1.

Best Mode for Carrying Out the Invention

The present disclosure provides a pharmaceutical composition for preventing or treating liver cancer recurrence or liver cancer metastasis, including an inhibitor of GULP1 expression or activity as an active ingredient.

Preferably, the inhibitor of GULP1 expression or activity may be selected from the group consisting of an antisense nucleotide, small interfering RNA (siRNA), short hairpin RNA (shRNA), compound, peptide, aptamer, and antibody that specifically bind to GULP1, but is not limited thereto.

Preferably, the liver cancer metastasis may be, but is not limited to, metastasis from a liver cancer to a lung cancer.

Preferably, the pharmaceutical composition may inhibit the binding of GULP1 and ARF6 and reduce protein stability of ARF6-GTP, but is not limited thereto.

Preferably, the pharmaceutical composition may inhibit an activity of β-catenin by inhibiting activation of ARF6, but is not limited thereto.

The pharmaceutical composition of the present disclosure may be prepared using pharmaceutically suitable and physiologically acceptable auxiliary agents in addition to the active ingredient, and solubilizers such as excipients, disintegrants, sweeteners, binders, coating agents, swelling agents, lubricants, glidants, or flavoring agents may be used as the auxiliary agent. The pharmaceutical composition of the present disclosure may be preferably formulated as a pharmaceutical composition by including one or more pharmaceutically acceptable carriers in addition to the effective ingredient for administration. In a composition formulated as a liquid solution, acceptable pharmaceutical carriers include those that are sterile and biocompatible, such as saline solution, sterile water, Ringer's solution, buffered saline, albumin injection solution, dextrose solution, maltodextrin solution, glycerol, ethanol, and mixtures of one or more of these components, and other conventional additives, such as antioxidants, buffers, and bacteriostatic agents, may be added as needed. In addition, diluents, dispersants, surfactants, binders, and lubricants may be additionally added to formulate into injectable formulations such as aqueous solutions, suspensions, and emulsions, pills, capsules, granules, or tablets.

The pharmaceutical formulation form of the pharmaceutical composition of the present disclosure may be granules, powder, coated tablets, tablets, capsules, suppositories, syrups, juices, suspensions, emulsions, drops or injectable liquids, and sustained-release formulations of active compounds. The pharmaceutical composition of the present disclosure may be administered in a conventional manner via intravenous, intraarterial, intraperitoneal, intramuscular, intraarterial, intraperitoneal, intrasternal, transdermal, intranasal, inhalation, topical, rectal, oral, intraocular, or intradermal routes. An effective amount of the active ingredient of the pharmaceutical composition of the present disclosure refers to an amount required for the prevention or treatment of a disease. Therefore, it may be adjusted according to various factors including the type of disease, severity of the disease, type and content of the active ingredient and other ingredients contained in the composition, type of formulation, and the patient's age, weight, general health, sex and diet, time of administration, route of administration, and secretion rate of the composition, treatment period, and drugs concurrently used. Although not limited to, for example, in the case of adults, when administered once to several times a day, the composition of the present disclosure may be administered at a dose of 0.1 ng/kg to 10 g/kg in the case of a compound, 0.1 ng/kg to 10 g/kg in the case of a polypeptide, protein or antibody, and 0.01 ng/kg to 10 g/kg in the case of an antisense nucleotide, siRNA, shRNA, or miRNA, when administered once to several times a day.

In addition, the present disclosure provides a health functional food composition for preventing or improving liver cancer recurrence or liver cancer metastasis, including an inhibitor of GULP1 expression or activity as an active ingredient.

The health functional food composition of the present disclosure may be provided in the form of powder, granules, tablets, capsules, syrup, or beverage, and the health functional food composition may be used together with other foods or food additives in addition to the active ingredient and used appropriately according to a conventional method. The amount of active ingredients mixed may be appropriately determined depending on its intended use, for example, prevention, health, or therapeutic treatment.

The effective dosage of the active ingredient contained in the health functional food composition may be used in accordance with the effective dosage of the pharmaceutical composition, but in the case of long-term intake for the purpose of health and hygiene or health control, it may be below the above range, and it is certain that the active ingredient may be used in an amount exceeding the above range since there is no problem in terms of safety.

There are no special restrictions on the types of the health foods, and examples include meat, sausage, bread, chocolate, candy, snacks, confectionery, pizza, ramen, other noodles, gum, dairy products including ice cream, various soups, beverages, tea, drinks, alcoholic beverages, and vitamin complexes.

In addition, the present disclosure provides a method of screening a treatment agent for liver cancer recurrence or liver cancer metastasis, including (1) contacting a test substance with isolated liver cancer cells; (2) measuring an expression or activity level of GULP1 in liver cancer cells that have come into contact with the test substance; and (3) selecting a test substance with the expression or activity level of GULP1 reduced compared to a control sample.

The term "test substance" used in referring to the screening method of the present disclosure refers to an unknown candidate substance used in screening to examine whether it affects the expression level of a gene or affects the expression or activity of a protein. The samples include, but are not limited to, chemicals, nucleotides, antisense RNA, siRNA (small interference RNA), and natural product extracts.

Modes for Carrying Out the Invention

Hereinafter, the present disclosure will be described in detail according to examples that do not limit the present disclosure. It should be noted that the following examples of the present disclosure are intended only to illustrate the present disclosure and do not limit or restrict the scope of the present disclosure. Therefore, it is interpreted that what may be easily inferred by an expert in the technical field to which the present disclosure pertains from the detailed description and examples of the present disclosure falls within the scope of the present disclosure.

<Experimental Example>

The following Experimental Examples are intended to provide Experimental Examples commonly applied to each Example according to the present disclosure.

1. Screening of specific candidate markers associated with liver cancer recurrence from genomic data of liver cancer patients who underwent hepatectomy.

To identify signatures of liver cancer recurrence, a total of three genomic data sets (GSE14520, GSE114564, TCGA_LIHC) were selected. The three datasets were used for analysis, by dividing patients into those who had recurrence (recurrence group) and those with no recurrence for more than two years (non-recurrence group) after hepatectomy.

2. Cell culture

Human normal hepatocytes (THLE-2) were obtained from ATCC (VA, USA), and human liver cancer cell lines (Hep-3B, Huh-7, PLC/PRF/5, SNU368, SNU398, SNU423) were obtained from the Korean Cell Line Bank (Seoul, Korea). THLE-2 were cultured in Bronchial Epithelium Basal Medium (BEBM, Lonza, MD, USA) containing 10% FBS, 5 ng/mL epidermal growth factor (EGF), and 70 ng/mL phosphoethanolamine. Liver cancer cell lines were cultured in DMEM medium containing 10% FBS and 100 units/mL penicillin-streptomycin (Invitrogen, CA, USA). All cultures were cultured under conditions where 5% carbon dioxide and a temperature of 37°C are maintained.

### 3. Measurement of GULP1 expression levels in cells via Western blotting

Cells were treated with protein lysis buffer to isolate proteins, followed by SDS-PAGE electrophoresis and Western blotting using GULP1 antibody (Cat #NBP1-84553, Novus biological).

### 4. Transfection

For siRNAs for transfection, control siRNA used was synthesized by Genolution (Seoul, Korea), and GULP1 siRNA used was AccuTarget™ Predesigned siRNA, a product manufactured and sold by Bioneer (Daejeon, Korea). The base sequence of control siRNA is shown in Table 1 below.

TABLE 1

| RNA duplexes | Sense sequence |
| --- | --- |
| Control siRNA | UUCUCCGAACGUGUCACGUUU |
| siGULP1 | CUGCAUAAGGACUACUCUU |
| siCTNNB1 | CCACAGCUCCUUCUCUGAGUGGUAA |
| siARF6 | GCGACCACUAUGAUAAUAUTT |

Transfection was performed using Lipofectamine 2000 (Invitrogen) reagent.

### 5. Analysis of cell growth

For cell growth analysis, cells were first seeded into a 24-well plate to about 30% confluence, transfected, and then treated with 0.5 mg/mL 3-(4,5-dimethylthiazol-2-yl)-2,5-diphenyltetrazolium bromide (MTT), followed by a reaction for 1 hour. Formazan crystals were dissolved in DMSO, and the absorbance was measured at 570 nm.
BrdU was added to the cells and reacted for 24 hours, followed by fixation of cells, and the DNA was denatured, treated with anti-BrdU antibody, and reacted with tetramethylbenzidine (TMB) substrate using HRP-conjugated secondary antibody, after which the absorbance was measured at 450 nm.

### 6. Culture of 3D cell spheres

Cell sphere culture was performed using methyl cellulose (Sigma-Aldrich).
For sphere culture, methyl cellulose was diluted to a 3% concentration in the medium, cells transfected with siGULP1 were injected and cultured for 1 hour at 37°C, and then culture was performed using a medium containing 10% FBS and 1% penicillin/streptomycin.
Few hours later, the cells forming the spheres were observed under a microscope.

### 7. Analysis of wound healing

Transfected cells were dispensed into a 6-well plate, and, when the cells reached 90-100% confluence, a scratch was made using a micropipette tip. Afterwards, the image of the area with the scratch was observed.

### 8. Collection of organs for verification and clinical information

After passing the IRB at Ajou University Hospital, research was proceeded by receiving 86 pairs of normal peripheral tissues and liver cancer tissues and their clinical information through the Human Genome Resource Bank.

### 9. RNA extraction from tissues

RNA from tissues was extracted using QIAzol Lysis Reagent (Cat#79306).

### 10. Measurement of expression levels of GULP1 in tissues via qRT-PCR analysis

cDNA synthesis was performed using the PrimeScript™ RT Master Mix (Perfect Real Time) (Cat #RR036A) (TaKaRa) kit.

Tissue RNA and RNase Free Water were combined to make 8 ul, and 2 ul of PrimeScript™ RT Master Mix was added to make a total of 10 ul for cDNA synthesis.

cDNA conditions were set as follows.

A. Stage 1: 37°C, 15 minutes
B. Stage 2: 85°C, 4°C after 5 seconds, -ing

qRT-PCR was performed using primers with the sequences below. The primers used here were purchased from M.biotech (Hanam, Korea). qRT-PCR was performed using 5 ul of qPCR Master Mix (2X, High ROX) (Gendepot, Cat #Q5602) in a total volume of 10 ul.

TABLE 2

| Gene | Accession No. | Forward sequence | Reverse sequence |
|------|--------------|------------------|------------------|
| GULP1 | NM_016315.4 | 5'- AACGGGACCTGTTTG G AGCA-3' | 5'- ACCCCTCCTGCATCTC ATCT-3' |
| HMBS | NM_001024382 .2 | 5'-GGAGGGCAGAAGGAAG AAAACAG-3' | 5'-CACTGTCCGTCTGTATG CGAG-3' |

qRT-PCR conditions were set as follows.

A. Stage 1: 95°C, 2 minutes
B. Stage 2: 95°C, 15 seconds
C. : 58°C, 34 seconds
D. : 72°C, 30 seconds
E. Repeat Stage 2 for 40 cycles

11. Immunofluorescence staining

Immunofluorescence staining was performed using β-catenin (Cell signaling Technology) antibody and 4',6-diamidino-2-phenylindole (DAPI, Thermo Fisher Scientific).

For immunofluorescence staining, cells transfected with siGULP1 were fixed with 4% paraformaldehyde (Sigma-Aldrich) for 10 minutes at room temperature, blocked with PBS containing 3% BSA at room temperature for 1 hour, and then washed twice with PBS. After washing, β-catenin antibody was diluted 1:100 in PBS containing 3% BSA, cultured overnight at 4°C, and washed twice with PBS. Next, Alexa fluor 488 goat anti-rabbit (Thermo Fisher Scientific) antibody was diluted 1:1000 in PBS containing 3% BSA, cultured for 1 hour at room temperature, and washed twice with PBS. After washing, DAPI was diluted in PBS at a concentration of 2 $\mu$g/mL, cultured at room temperature for 10 minutes, and washed once with PBS. Immunofluorescently stained cells were observed under a fluorescence microscope.

12. Analysis of protein expression through immunoblotting

Proteins were extracted from siGULP1-transfected cells using cell lysate. BCA assay was performed to measure the concentration of extracted protein. The extracted protein was diluted to a concentration of 20 $\mu$g/ml to perform immunoblotting.

The bound proteins separated by SDS-PAGE were transferred to a nitrocellulose membrane (NC membrane) to perform Western blotting, and the NC membrane with the protein transferred was blocked in 5% skim milk for 1 hour at room temperature. The NC membrane was washed twice for 5 minutes each using TBS-T solution, followed by an overnight reaction between the washed NC membrane and primary antibodies at 4°C. After washing three times with TBS-T for 5 minutes, a reaction was performed with secondary antibodies conjugated with horseradish peroxidase (HRP) at room temperature for 1 hour, followed by washing four times with TBS-T for 5 minutes, then a reaction was carried out with ECL substrate (Thermo Fisher Scientific), and protein expression was observed using LAS4000 imaging equipment.

13. Analysis of protein binding via immunoprecipitation

siGULP1-transfected cells were lysed with cell lysis buffer (Cell lysis buffer: 1% triton X-100, 150 mM NaCl, 50 mM Tris-Cl (pH 7.5), 0.1% SDS, 1% NP-40, 1 mM PMSF), and to obtain the protein supernatant from the cell lysate, centrifugation was carried out at 12,000 rpm at 4°C for 10 minutes. To transfer the supernatant to a new tube and perform immunoprecipitation analysis, the cell lysate was treated with anti-β-catenin and anti-GULP1 antibodies (1 μg) and reacted in PBS for 1 hour, and then protein A/G-agarose beads were added and reacted for 24 hours at 4°C. The beads were washed three times with PBS containing 1 mM DTT and boiled for 5 minutes along with 2X protein loading dye (25% SDS, 62.5 mM Tris-HCl (pH 6.8), 25% Glycerol, and 0.01% BROMOPHENOL BLUE). The samples were separated on electrophoresis via SDS-PAGE.

The bound proteins separated by SDS-PAGE were transferred to a nitrocellulose membrane (NC membrane) to perform Western blotting, and the NC membranes with the protein transferred were blocked in 5% skim milk for 1 hour at room temperature. The NC membrane was washed twice for 5 minutes each using TBS-T solution, and the washed NC membrane was reacted with primary antibodies overnight at 4°C. The NC membrane was washed twice for 5 minutes each using TBS-T solution, and the washed NC membrane was reacted with primary antibodies overnight at 4°C. After washing three times with TBS-T for 5 minutes each and then reacting with secondary antibodies conjugated with horseradish peroxidase (HRP) at room temperature for 1 hour, washing was carried out four times with TBS-T for 5 minutes each, followed by observation with LAS4000 imaging equipment using enhanced chemo-luminal (ECL).

14. Chromatic immunoprecipitation

Pre-clearing and sedimentation beads were prepared. Pre-clearing beads were prepared by culturing 15 μl protein A/G agarose slurry (Santacruz) having 2 μl of purified rabbit IgG (Thermo Fisher Scientific) in 120 μl of ChIP Dilution Buffer per sample for 1 hour at room temperature. After going through two washing steps using 1 volume of ChIP dilution buffer each, beads were resuspended in 85 μl of ChIP dilution buffer. 15 μl of agarose A/G beads for precipitation were blocked in 1 ml of ChIP dilution buffer containing 5 mg/ml BSA (Santacruz) and 100 μg/ml pre-warmed salmon sperm DNA (Invitrogen) at 95°C for 10 minutes and on ice for 5 minutes. The resuspended pellet was cultured with rotation at 4°C for 4-5 hours, washed three times, and mounted in a double volume of ChIP dilution buffer.

After collecting approximately $1 \times 10^7$ cells transfected with siGULP1 with a viability of more than 97%, 3.7% formaldehyde was added, and fixation was carried out by culturing at room temperature for 10 minutes. After fixation was stopped by adding 1x glycine solution, washing was performed twice with ice-cold PBS followed by centrifugation at 12,000 rpm for 3 minutes, and the pellet was resuspended in 1 ml of PBS containing the protease inhibitor Roche complete (Roche Diagnostics GmbH). The suspension was pelleted by centrifugation at 14,000 rpm at 4°C for 5 minutes, and the supernatant was removed.

Pellet lysis was performed by adding Roche complete to 1 ml cell lysis buffer, followed by culture on ice for 10 minutes. After centrifugation at 13,000 rpm for 5 minutes at 4°C, the nuclear pellet was resuspended in 300 μl of nuclear lysis solution, sonicated for 15 seconds at Output 5 and 90% duty cycle, and cultured on ice for 2-3 minutes, followed by 6 cycles of sonication (Branson Sonifier B15). After adding 700 μl of nuclear lysis solution to the sonicated nuclei, the nuclei were centrifuged at 13,000 rpm at 4°C for 15 minutes. The supernatant was transferred to a new tube to be stored at -80°C until use. The grade of sonication after protein digestion was tested on agarose gel.

After chromatin was cultured with rotation at 4°C for 2 hours, centrifugation was followed at 13,000 rpm for 10 minutes at 4°C, and then pre-clearing was performed with 80 μl of protein A/G agarose slurry prepared. The supernatant was transferred to a new tube, protein concentration was measured using a BCA assay (Thermo Fisher Scientific), and 25-100 μg of chromatin per immunoprecipitate (IP) was loaded in 200 μl nuclear lysis solution, which was then added to 300 μl ChIP dilution buffer containing 3 μg of antibody. The immunoprecipitate was cultured overnight at 4°C with rotation. Undiluted input samples were stored at -20°C. After overnight culture, 40 μl of blocked protein A/G agarose beads were added to the IP solution and cultured with rotation at 4°C for 1 hour. The sediment was washed twice with 1 ml of low-salt washing buffer, once with high-salt washing buffer, once with LiCl washing buffer, and once with TE washing buffer on a rotation platform for 5 minutes. After each washing, centrifugation was performed at 6,000 rpm at 4°C for 30 seconds. After the final washing step, the beads were centrifuged at 6,000 rpm for 1 minute, and the bead pellet was combined with 200 μl of IP elution buffer while simultaneously filling 25-100 μg of chromatin input sample with IP elution buffer up to 200 μl, followed by culture at 65°C with shaking for 30 minutes. Next, 0.5 μl of RNAse DNAse free (Roche) was added to each tube followed by culture at 37°C for 30 minutes, 10 μl of 4 M NaCl (final concentration of 0.2 M) and 2 μl of proteinase K (final concentration of 100-200 μg/ml, Takara) were added to the reaction sample for final protein digestion and cultured at 65°C for 1.5 hours, and then DNA was purified using a PCR purification kit (QIAGEN).

15. Mouse subcutaneous transplantation model

For xenograft tumor formation assay, $5 \times 10^5$ transfected cells were mixed with serum-free media and 20% Matrigel, and 100 μl of the cell suspension was injected subcutaneously into 6-week-old BALB/c nude mice.

The mice were observed for tumor formation at the injection site three times a week. Each experimental group consisted of four mice, and for tumor growth, the tumor size was measured in three orthogonal directions using digital

calipers.

$$\text{Tumor volume was calculated as } 0.5 \times \text{length (L)} \times \text{width}^2 \text{ (W}^2).$$

16. Mouse tail vein injection model

For the tail vein injection model to find the metastatic ability of liver cancer cells, $3 \times 10^6$ transfected RAS-NIH-3T3 cells were mixed with serum-free media, and 200 μl of the cell suspension was injected into the tail vein of 6-week-old athymic mice.

The body weights of the mice were measured twice a week, and after two weeks, the mice were sacrificed to observe liver cancer cells that had metastasized to the nodules formed in the lungs.

17. Mouse orthotopic transplantation model

To identify the recurrence ability of liver cells, the skin and peritoneum of the mouse were incised to expose the left lobe of the liver to the outside, and $5 \times 10^5$ transfected Huh-7 cells were mixed with serum-free media and 50% Matrigel, and 10 μl of the cell suspension was injected into the left lobe of the liver of the 6-week-old BALB/c nude mouse using a Hamilton micro syringe.

After 18 days, the upper abdominal wall of the mouse was resected again to expose the left lobe of the liver where the cell suspension was injected to observe tumor formation, the area of the liver where the tumor had formed was resected, then the left lobe of the liver was placed back into the body, and the incision site was sutured.

Two weeks after liver tumor resection, mice were sacrificed and the number of recurrent tumors in the left lobe of the liver as well as the size and weight of the tumors were determined.

Transfected cells were injected into the mouse liver to detect the tumors tissues by staining the tumor formed and the tumor recurred after tumor resection using H&E, and, after liver tumor resection, changes in the expression of related markers were observed in the recurred tumors using IHC.

<Example 1> Identification of specific candidate markers associated with liver cancer recurrence and evaluation of clinical relevance related to recurrence from genomic data of liver cancer patients who underwent hepatectomy.

To identify liver cancer recurrence signatures, a total of three genomic data sets (GSE14520, GSE114564, TCGA_LIHC) were selected. The three datasets were used for analysis, by dividing patients into those with recurrence (recurrence group) and those without for more than two years (non-recurrence group) after hepatectomy.

As a result of analysis, GULP1 showing higher expression in the recurrence group compared to the non-recurrence group was identified in all three datasets.

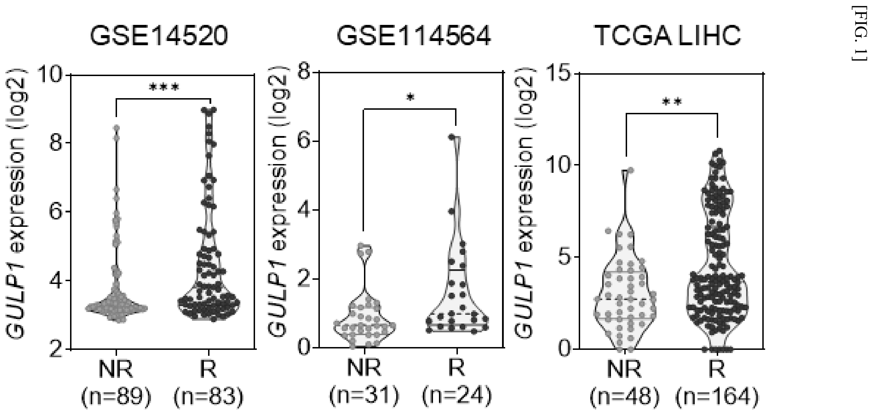

It was determined that GULP1 has statistically significantly higher expression in the recurrence group compared to the non-recurrence group in all three datasets (FIG. 1).

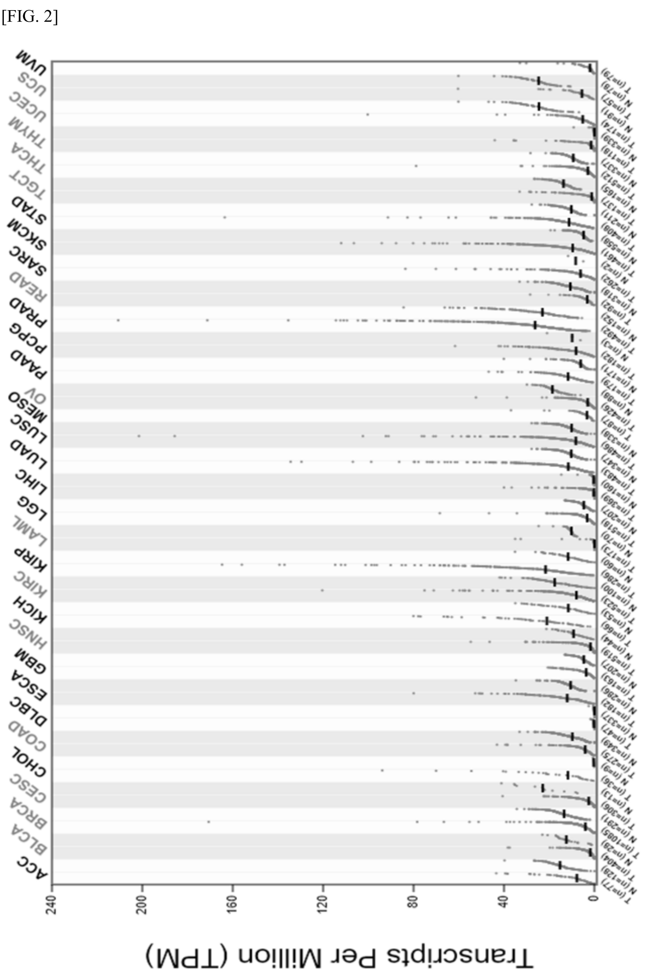

When the expression of GULP1 was analyzed for a total of 33 carcinomas using TCGA Pan-cancer data, expression of GULP1 was rather suppressed in tumors in most carcinomas (green), which is predicted to be a tumor suppressor (FIG. 2).

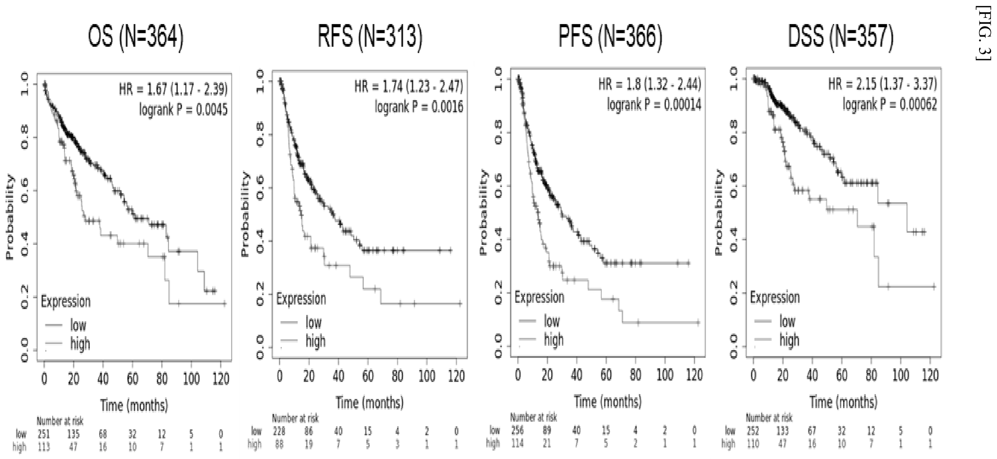

However, in liver cancer, it was analyzed that the prognosis was all worse in OS, RFS, PFS, and DSS in the patient group with high expression of GULP1, which was expected that GULP1 would play a major role in the progression of liver cancer (FIG. 3).

<Example 2> Verification of an ability to control liver cancer cell growth by GULP1

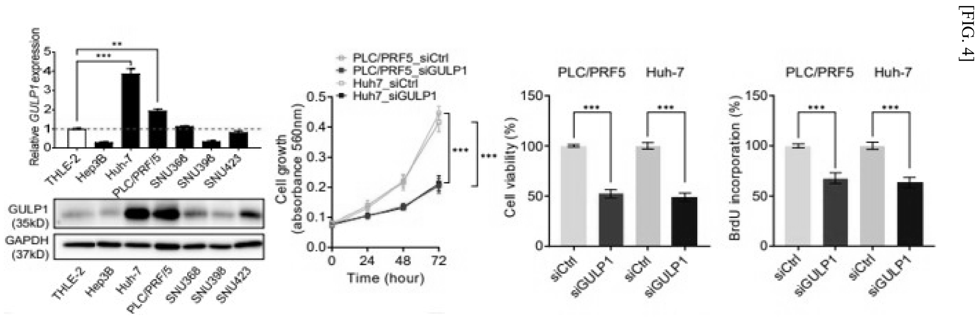

Two liver cancer cell lines (Huh-7, PLC/PRF/5) with increased expression of GULP1 were selected to determine whether it affects the growth ability of liver cancer. First, when GULP1 was knocked down to perform MTT and BrdU assays, it was found that the growth of liver cancer cells was reduced (FIG. 4).

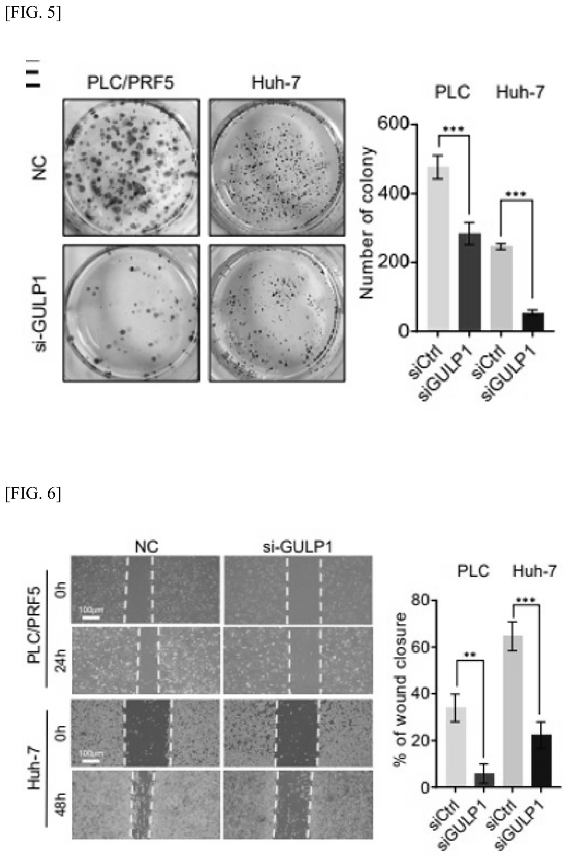

In addition, when GULP1 was knocked down to analyze the effect on the colony forming ability, it was revealed that colony formation of cancer cells was significantly reduced compared to the control group (FIG. 5).

<Example 3> Verification of functions in controlling liver cancer cell metastasis by GULP1

To determine whether GULP1 is involved in liver cancer cell metastasis, a wound healing assay was performed. When the expression of GULP1 was reduced, it was found that the wound healing ability was reduced compared to the experimental control group (FIG. 6).

<Example 4> Identification of cell sphere formation and metastatic ability by GULP1

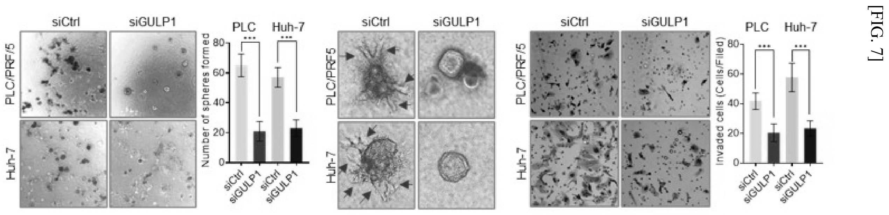

To determine whether GULP1 is involved in the growth and metastasis of liver cancer cells, three-dimensional sphere culture and cell metastasis assays were performed. Knockdown of GULP1 by siGULP1 transfection inhibited the sphere forming ability of liver cancer cells, and, as a result of observing the formed spheres at high magnification, cell outgrowth around the formed spheres was inhibited, with a decrease in the degree of cell invasion (FIG. 7).

<Example 5> Identification of the mechanism of GULP1

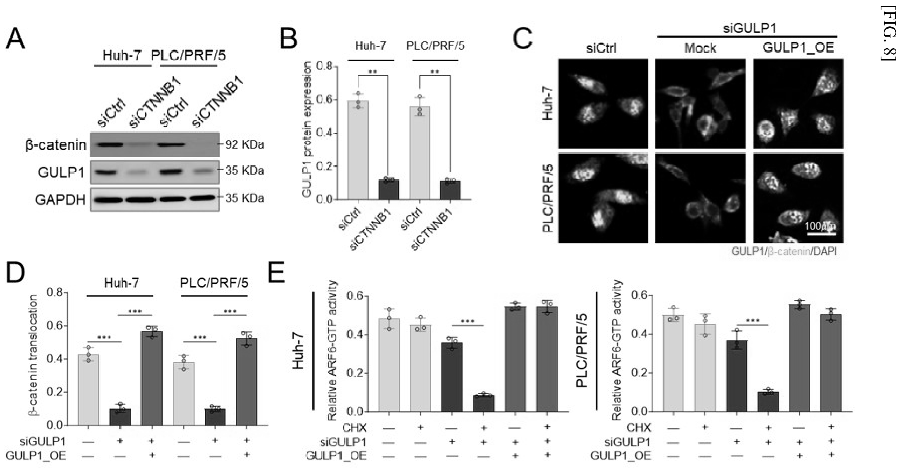

To clarify the mechanistic role of GULP1 in HCC progression, its effect on β-catenin signaling was investigated. In evaluation of liver cancer cell lines, observation was conducted for β-catenin inhibition causing a significant decrease in GULP1 protein levels (FIGS. 8A and 8B).

The effect of changes in the GULP1 expression on intracellular β-catenin transport was clearly observed using fluorescence microscopy (FIG. 8C and FIG. 8D).

To determine the mechanism by which GULP1 regulates β-catenin transport, investigation was conducted on the interaction between GULP1 and ARF6-GTP. In the identification of the role of GULP1 in stabilizing ARF6 activity, a decrease in GULP1 or changes in ARF6-GTP was not observed. However, when GULP1 expression was reduced under conditions where protein synthesis was inhibited by cycloheximide (CHX), ARF6-GTP levels were suppressed. In contrast, the rescue effect by ARF6-GTP was observed upon inhibition and overexpression of GULP1 in the presence of CHX (FIG. 8E).

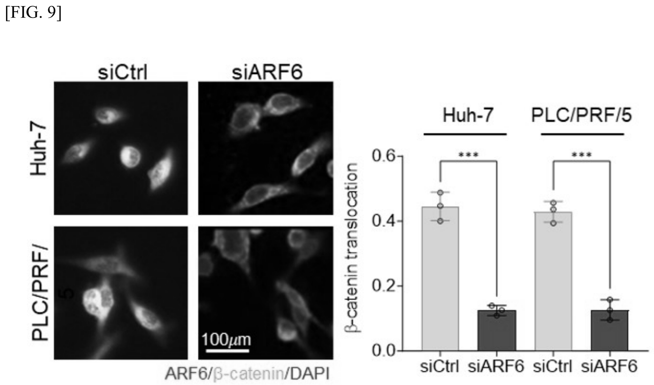

These results suggest that GULP1-mediated β-catenin translocation is dependent on ARF6, which is an important regulatory mechanism in cellular processes. These results reveal that GULP1-mediated β-catenin translocation is dependent on ARF6, as observed that β-catenin translocation was inhibited through suppression of ARF6 (FIG. 9).

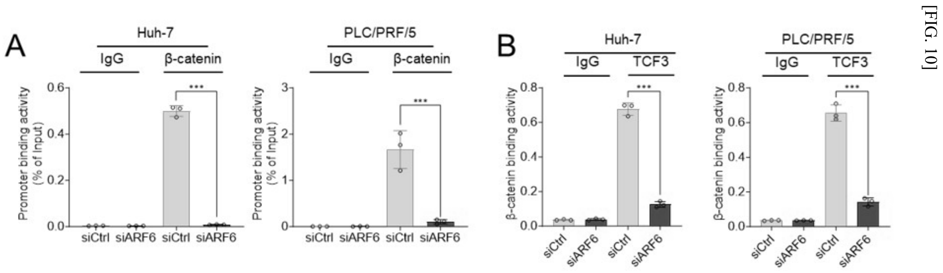

Investigation was conducted on the effect of ARF6 regulation on the interaction between β-catenin and TCF3 and the expression of GULP1. Referring to the sequence of TCF3 binding motifs, the GULP1 promoter region (chr 2: 189,158,687-189,158,701) was predicted to be the binding site. The results of Chromatin Immunoprecipitation (ChIP) experiments showed that ARF6 reduction lowered the binding of β-catenin and TCF3 to the GULP1 promoter region (FIG. 10A and FIG. 10B).

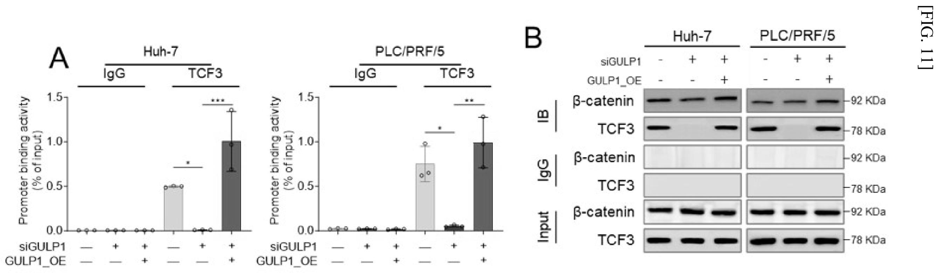

Additionally, when GULP1 expression was regulated, a change in the degree of binding of TCF3 to the GULP1 promoter region was observed (FIG. 11A). This suggests that these proteins form a complex involving direct binding between β-catenin and TCF3 in the GULP1 promoter region, as evidenced by the result from the co-immunoprecipitation (co-IP) (FIG. 11B).

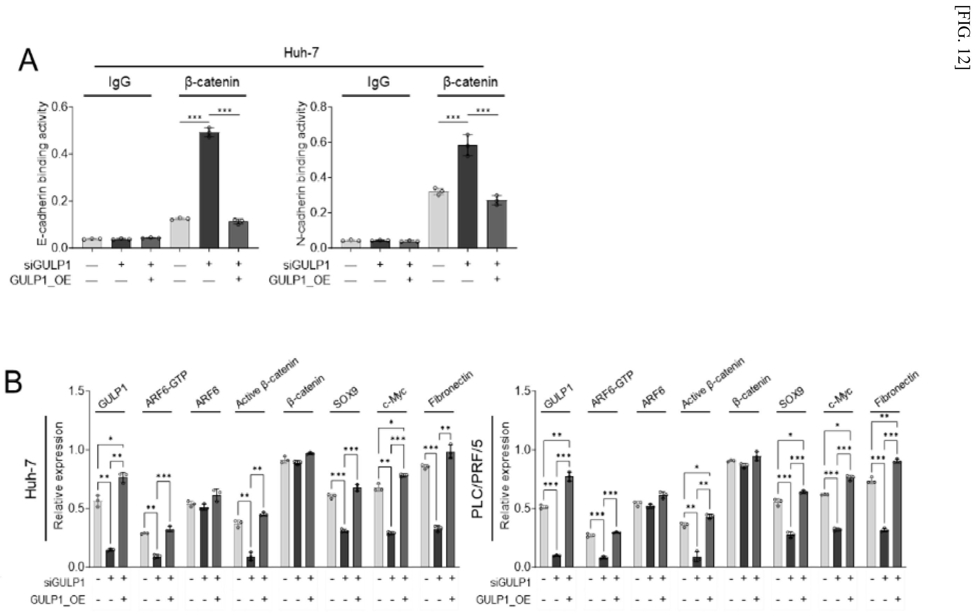

GULP1 was shown to regulate β-catenin interactions with key associated factors, N-cadherin and E-cadherin (FIG. 12A). This function of GULP1 affects downstream factors of β-catenin, as well as SOX9, c-Myc, and fibronectin, presenting a feedback mechanism in β-catenin signaling that may significantly affect HCC progression (FIG. 12B).

<Example 6> Verification of a cancer cell control ability by GULP1 through a mouse subcutaneous transplantation model

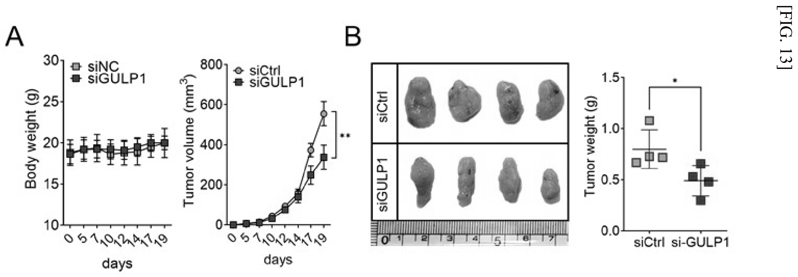

As a result of observing tumor formation in mice subcutaneously injected with the Huh-7 liver cancer cell line with reduced expression of GULP1, it was found that the size and weight of the tumors were significantly reduced compared to the experimental control group, which showed the same result as the test tube experiment (FIG. 13).

<Example 7> Identification of a metastatic and recurrence ability of GULP1 using a mouse model.

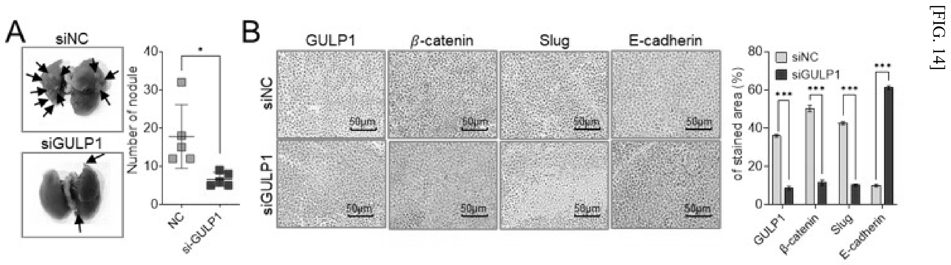

To determine whether GULP1 is involved in liver cancer metastasis, when the metastatic ability of GULP1 was identified by tail vein injection, there was a decrease in the number of nodules formed in the lungs of mice in which ras-NIH-3T3 cells transfected with siGULP1 were injected into the tail vein, showing the metastatic ability of GULP 1 (FIG. 14).

In addition, as a result of observing the expression of related markers by IHC in mouse tumors injected subcutaneously with Huh-7 cells transfected with siGULP1, the expression of GULP1 and β-catenin was decreased, and the expression of E-cadherin, an EMT marker, was increased, while that of Slug was decreased (FIG. 14).

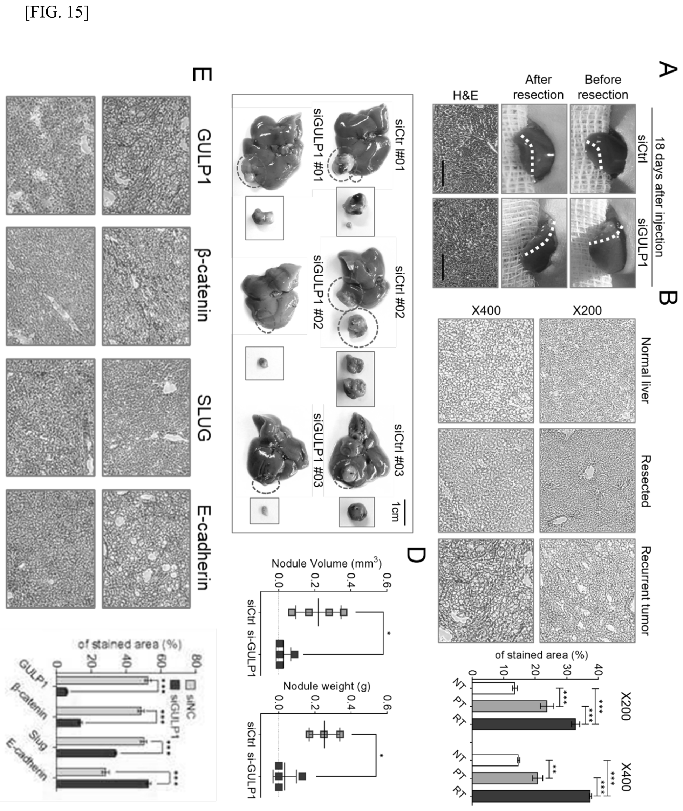

To identify the possibility of liver cancer recurrence by GULP1, when Huh-7 cells transfected with siGULP1 were injected into the liver of mice in an orthotopic mouse model and then the formed tumors were resected to observe the recurred tumors, it was found that the tumor size and weight were reduced, and the IHC results for related markers showed that the expression of GULP1 and β-catenin decreased, and the expression of E-cadherin, an EMT marker, increased, while that of Slug decreased (FIG. 15).

As a result of detecting expression of GULP1 by injecting transfected Huh-7 into the liver of mice and then resecting the formed tumor tissues and tumors to perform IHC on the recurred tumor tissues, the expression of GULP1 increased in the resected tumor tissues compared to the normal liver tissue, and the expression was found to be increased further in the recurrent tumor tissues compared to the resected tumor tissues (FIG. 15).

While a specific part of the present disclosure has been described in detail above, it is clear for those skilled in the art that this specific description is merely preferred example embodiments, and the scope of the present disclosure is not limited thereby. In other words, the substantial scope of the present disclosure is defined by the appended claims and their equivalents.

**Claims**

1.  A pharmaceutical composition for preventing or treating liver cancer recurrence or liver cancer metastasis, comprising an inhibitor of GULP1 expression or activity as an active ingredient.

2.  The pharmaceutical composition of claim 1, wherein the inhibitor of GULP1 expression or activity is any one selected from the group consisting of an antisense nucleotide, small interfering RNA (siRNA), short hairpin RNA (shRNA), compound, peptide, aptamer, and antibody that specifically bind to GULP1.

3.  The pharmaceutical composition of claim 1, wherein the liver cancer metastasis is metastasis from a liver cancer to a lung cancer.

4.  The pharmaceutical composition of claim 1, wherein the pharmaceutical composition inhibits binding of GULP1 and ARF6 and reduces protein stability of ARF6-GTP.

5.  The pharmaceutical composition of claim 1, wherein the pharmaceutical composition inhibits an activity of $\beta$-catenin by inhibiting activation of ARF6.

6.  A health functional food composition for preventing or improving liver cancer recurrence or liver cancer metastasis, comprising an inhibitor of GULP1 expression or activity as an active ingredient.

7.  A method of screening a treatment agent for liver cancer recurrence or liver cancer metastasis, the method comprising:

    (1) contacting a test substance with isolated liver cancer cells;
    (2) measuring an expression or activity level of GULP1 in liver cancer cells that have come into contact with the test substance; and
    (3) selecting a test substance with the expression or activity level of GULP1 reduced compared to a control sample.

[FIG. 1]

[FIG. 2]

Transcripts Per Million (TPM)

[FIG. 3]

[FIG. 4]

[FIG. 5]

[FIG. 6]

[FIG. 7]

GULP1/β-catenin/DAPI

[FIG. 8]

[FIG. 9]

[FIG. 10]

[FIG. 11]

[FIG. 12]

[FIG. 13]

[FIG. 14]

[FIG. 15]

## INTERNATIONAL SEARCH REPORT

| International application No. |
|---|
| **PCT/KR2024/005599** |

### A. CLASSIFICATION OF SUBJECT MATTER

**A61K 31/713**(2006.01)i; **A61P 35/04**(2006.01)i; **A23L 33/13**(2016.01)i; **G01N 33/50**(2006.01)i; **C12Q 1/6886**(2018.01)i; **A61K 48/00**(2006.01)i

According to International Patent Classification (IPC) or to both national classification and IPC

### B. FIELDS SEARCHED

Minimum documentation searched (classification system followed by classification symbols)

A61K 31/713(2006.01); C12N 15/11(2006.01); C12Q 1/6886(2018.01); G01N 33/50(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: GULP1, 간암(hepatic cancer), 간섭RNA(RNA interference, RNAi), 재발 (recurrence), 전이(metastasis)

### C. DOCUMENTS CONSIDERED TO BE RELEVANT

| Category* | Citation of document, with indication, where appropriate, of the relevant passages | Relevant to claim No. |
|---|---|---|
| Y | QIAN, X. et al. Recurrence risk of liver cancer post-hepatectomy using machine learning and study of correlation with immune infiltration. Frontiers in Genetics. 2021, vol. 12, thesis no.:733654, pp. 1-14.<br>See abstract; and pages 11-12. | 1-7 |
| Y | KR 10-2019-0049582 A (INDUSTRY-ACADEMIC COOPERATION FOUNDATION, YONSEI UNIVERSITY) 09 May 2019 (2019-05-09)<br>See abstract; and claims 1, 3-4, 6 and 8. | 1-7 |
| Y | CN 112831562 A (ZHEJIANG UNIVERSITY OF SCIENCE AND TECHNOLOGY) 25 May 2021 (2021-05-25)<br>See abstract; table 1; and claims 1-8. | 1-7 |
| A | DU, B. et al. A novel signature based on microvascular invasion predicts the recurrence of HCC. Journal of translational medicine. 2020, vol. 18, thesis no.:272, pp. 1-10.<br>See entire document. | 1-7 |

☑ Further documents are listed in the continuation of Box C.    ☑ See patent family annex.

| | | |
|---|---|---|
| * | Special categories of cited documents: | |
| "A" | document defining the general state of the art which is not considered to be of particular relevance | "T" later document published after the international filing date or priority date and not in conflict with the application but cited to understand the principle or theory underlying the invention |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" document member of the same patent family |
| "P" | document published prior to the international filing date but later than the priority date claimed | |

| Date of the actual completion of the international search | Date of mailing of the international search report |
|---|---|
| **14 August 2024** | **16 August 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
| --- |
| **PCT/KR2024/005599** |

### C. DOCUMENTS CONSIDERED TO BE RELEVANT

| Category* | Citation of document, with indication, where appropriate, of the relevant passages | Relevant to claim No. |
| --- | --- | --- |
| A | KR 10-2020-0119538 A (THE CATHOLIC UNIVERSITY OF KOREA INDUSTRY-ACADEMIC COOPERATION FOUNDATION) 20 October 2020 (2020-10-20) See entire document. | 1-7 |

Form PCT/ISA/210 (second sheet) (July 2022)

## INTERNATIONAL SEARCH REPORT
### Information on patent family members

| Patent document cited in search report | | | Publication date (day/month/year) | Patent family member(s) | | | Publication date (day/month/year) |
|---|---|---|---|---|---|---|---|
| KR | 10-2019-0049582 | A | 09 May 2019 | KR | 10-2194537 | B1 | 23 December 2020 |
| CN | 112831562 | A | 25 May 2021 | None | | | |
| KR | 10-2020-0119538 | A | 20 October 2020 | KR | 10-2021-0056959 | A | 20 May 2021 |
| | | | | KR | 10-2344598 | B1 | 29 December 2021 |

Form PCT/ISA/210 (patent family annex) (July 2022)